(19)

Europäisches Patentamt
European Patent Office
Office européen des brevets

(11) **EP 3 231 508 A1**

(12) **EUROPEAN PATENT APPLICATION**

(43) Date of publication:
**18.10.2017 Bulletin 2017/42**

(21) Application number: **17159647.1**

(22) Date of filing: **07.03.2017**

(51) Int Cl.:
*B01J 19/00* (2006.01)       *C12Q 1/68* (2006.01)
*G01N 33/50* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **08.03.2016   US 201662305122 P**

(71) Applicant: **Northeastern University**
**Boston, MA 02115 (US)**

(72) Inventors:
• **SU, Ming**
  **Newton, MA 02465 MA (US)**
• **MA, Liyuan**
  **Newton, MA 02465 MA (US)**

(74) Representative: **Høiberg P/S**
**Adelgade 12**
**1304 Copenhagen K (DK)**

<u>Remarks:</u>
Claims filed after the date of filing of the application (Rule 68(4) EPC).

(54) **DISPOSABLE SINGLE CELL ARRAY FOR PERSONALIZED DIAGNOSTICS**

(57)     Paper-based single cell arrays are provided, as well as methods of making and using the arrays. The invention provides a low cost, high-throughput platform to detect and quantify different types of DNA damage at point-of-care without expensive equipment or highly trained personnel. Ordinary paper can be covered with multiple layers of common printing ink and micro-patterned to form discrete and ordered arrays capable of binding a single cell, which are then lysed and imaged. The platform allows quick and inexpensive testing of multiple anti-cancer treatment options for a particular patient. The invention can make cancer treatment personalized and more effective, even in low-resource settings.

FIG. 1A

# FIG. 1B

Modified Stamp

Stamp printing

Gel capping

Multi-sample loading

Halo assay

Halo assay

## Description

CROSS REFERENCE TO RELATED APPLICATIONS

This application claims the priority of U.S. Provisional Application No. 62/305,122 filed 8 March 2016 and entitled "Disposable Single Cell Array for Personalized Diagnostics", which is hereby incorporated by reference in its entirety.

STATEMENT REGARDING FEDERALLY SPONSORED RESEARCH OR DEVELOPMENT

This invention was developed with financial support from Grant No. 1DP2EB016572 from the National Institutes of Health. The U.S Government has certain rights in the invention.

BACKGROUND

Cancer is the uncontrolled growth of cells coupled with malignant invasion and metastasis. It is a global health issue that causes millions of deaths worldwide every year, 70% of which occur in low- and middle-income countries. Low-cost chemotherapy and affordable radiation therapy (based on cobalt 60 source) are often used to treat patients and prevent cancer cells from growing. A common feature of many treatments against cancer, including varied chemotherapy drugs and radiation therapy, is that DNA damage is involved as a key element. The drugs or radiation conditions, such as total dose and dose pattern, used in the clinic today were selected against stringent processes that involved a wide range of cell lines, animal models and human subjects. However, individual patient responses to the same drug or radiation condition remain largely different even for cancers of identical tissue origin and histology. Consequently, whereas current treatments benefit some patients, others receive no benefit or suffer with adverse reactions. Instead of trying to find an ideal drug or radiation condition that can be universally applied to all cancer patients or all patients of one type of cancer, the efficacies of existing drugs or radiation conditions could be examined on tumor cells extracted from patients at clinical sites before treatment can be prescribed. It is expected that personalized cancer treatment tailored to each patient's tumor can improve outcome, avoid unnecessary treatment and reduce healthcare costs. However, subjecting small tumor tissue samples obtained from a patient to a battery of screening assays is expensive, and too time- and resource-demanding to be realistic. Performing genetic tests on patients could enhance cancer treatment by dividing patients into subgroups, but such tests are complex and expensive at present. Moreover, these tests are intrinsically limited to evaluating genetic variation alone, without accounting for phenotypical variance arising from epigenetics and other environmental factors.

A variety of *in vitro* assays can be used to assess efficacies of drugs or radiation conditions, but the need for a strategy that can predict patient response in clinics is largely unmet. Existing assays based on cell growth or cell death, such as traditional tumor clonogenic assay, [$^3$H] thymidine incorporation and ATP (adenosine 5'-triphosphate) bioluminescence assay need a long period of cell expansion, during which many changes can occur under culture conditions. Moreover, growth inhibition assays can promote single clones, and thus may not reflect true tissue responses. Standard macroscopic tissue-based histological assays are limited by sample preparation; they rely on samples from patients being cut into thin sections, but such practice will unavoidably generate a large portion of truncated and overlapped cells that should not be scored, because truncations cause under-count, and overlaps cause over-count. Identification of intact isolated cells is often done manually, and is subjective and laborious. As a result, only a small number of cells (~50) can be scored for each sample. Considering that a small tumor of 1 cm$^3$ volume contains 1 billion cells, the small number of cells examined in conventional assays is insufficient to quantify tissue response. Flow cytometry can detect specific proteins (phosphorylation of histone) associated with double strand breaks of DNAs, but it is expensive and requires highly trained personnel, being mostly limited to research environments. Microfluidic cytometry is cheaper, but it needs extensive training of personnel to handle multiple components such as fluorescence labeling, liquid delivery and optical readout. Most importantly, both flow cytometry and its microfluidic derivative are inappropriate to detect a wide variety of additional types of damages such as single-strand breaks, inter-strand crosslinks and base damages.

Single cell-based DNA damage assay can reveal effective sensitivity profiles of a drug or radiation, and test cell responses without population averaging. Comet assay (single cell gel electrophoresis) can be used to detect single and double strand DNA breaks by assessing size and fragmentation patterns of DNAs from comet tails, but comet assay is limited by low throughput and poor reproducibility between end-user groups. The random 3D distribution of cells in a porous gel requires user to constantly scan multiple fields of focus to find cells at different heights and locations. Cells may form unanalyzable clumps, random debris and overlapping comets, which lead to loss of valuable information especially from rare cells. A comet chip has been used to solve the random distribution issue by trapping cells inside microwells at the same height and same location, but each comet has to be individually analyzed due to complicated shape of a comet tail, which requires intensive and potentially biased user intervention to identify the head and tail of a comet for each cell. Even with measured head/tail sizes, quantifying DNA damage with comet assay is intrinsically empirical, because the comet shape is determined by many factors such as electric field strength, gel concentrations, degree of gelation and buffer conditions.

Comet assay also takes a long time (~2 h) to pull DNA molecules out of cells, which is not suitable for point-of-care application.

DNA damage can also be assessed with halo assays. This approach takes advantage of the intrinsic diffusion of damaged DNA fragments out of cells at room temperature, which form a halo inside a homogeneous gel environment, wherein the halo radius is proportional to level of DNA damage. As drug/radiation causes damage at equal probability along the DNA chain, the halo radius is proportional to the amount of damaged DNAs. The self-diffusion time of DNA is surprisingly shorter than that of comet assay at the same level of damage, likely due to the fact that DNA fragments are not pulled out of the cell along one particular direction; while in comet assay, some fragments have to pass through high density nuclei. After fluorescent staining of DNAs, a halo (low fluorescent intensity) will form around a core (high fluorescent density) from each cell, wherein the symmetrical shape of the halo will greatly facilitate accurate determination of DNA damage. However, since cells are randomly dispersed inside an agarose gel, the random 3D distributions of cells and halos form unanalyzable clumps, as well as overlapping cells and halos.

There is ongoing need for inexpensive, easy-to-use, clinically applicable methods of detecting and quantifying DNA damage that can be employed even at low-resource, point-of-care settings.

SUMMARY OF THE INVENTION

The invention provides single cell arrays that can be made on ordinary printer paper or other disposable substrates for use in medical diagnostics and treatment regimens. In particular, the invention can be used to assess the cellular toxicity of various agents, which can be used to quickly and inexpensively determine an individual patient's response to different cancer treatments. The array can be made using a common inkjet printer and micro-contact printing to form micro-patterned single cell arrays. The method of production is simple and low cost, and it is broadly applicable to a wide variety of bioassays.

One aspect of the invention is a single-cell array precursor, comprising a paper substrate; a polyanion layer deposited on a side of the paper substrate; and a patterned array of polycation regions deposited on the polyanion layer.

In some embodiments, the polyanion layer includes printing ink. In some embodiments, the polyanion layer includes 3 to 5 layers of printing ink. In some embodiments, the printing ink is liquid ink or toner.

In preferred embodiments, the polycation regions include polydiallyldimethyl ammonium chloride (PDAC). In some embodiments, the patterned array includes polycation regions having a size of about 5 $\mu$m to about 40 $\mu$m. In other embodiments, the patterned array includes polycation regions having a size of about 10 $\mu$m

to about 20 $\mu$m. In a typical embodiment, the patterned array includes polycation regions having a size that allows attachment of a single cell.

Another aspect of the invention is single-cell array, comprising the precursor of claim 1 and further comprising a plurality of cells individually attached to said polycation regions. In a typical embodiment, each polycation region is bound to at most a single cell. In some embodiments, the array further includes an agarose layer embedding the cells.

Yet another aspect of the invention is method of quantifying DNA damage. The method includes: (a) providing the single-cell array precursor of claim 1; (b) contacting the precursor with a plurality of cells, whereby each cell is individually attached to a polycation region; (c) embedding the cells in an agarose layer; and (d) treating the cells with an alkaline solution. In preferred embodiments, each polycation region is bound to at most a single cell.

In some embodiments, DNA damage is quantified by calculation of the nuclear diffusion factor. In some embodiments, DNA damage quantification is automated.

In certain embodiments, step (b) further includes exposing cells to a DNA-damaging agent. In certain embodiments, step (d) further includes exposing cells to a DNA-damaging agent. In some embodiments, the method further includes the step of staining the cells with a fluorescent DNA dye. In some embodiments, the method further includes the step of imaging the cells. In some embodiments, cells are imaged using fluorescence microscopy. In some embodiments, cells are imaged using a mobile phone camera.

Another aspect of the invention is a method of making a single cell array precursor. The method includes (a) providing a paper substrate, a polyanionic substance, and a polycationic substance; (b) coating the paper with the polyanionic substance to form one or more polyanion layers on a side of the paper substrate; and (c) coating regions of the polyanion layer with a polycationic substance to form an array of polycation regions, the regions sized to allow attachment of a single cell to each region.

Another aspect of the invention is a method of making a single cell array. The method includes: (a) providing a paper substrate, a polyanionic substance, a polycationic substance, and a plurality of single cells; (b) coating the paper with the polyanionic substance to form one or more polyanion layers on a side of the paper substrate; (c) coating regions of the polyanion layer with a polycationic substance to form an array of polycation regions, the regions sized to allow attachment of a single cell to each region; and (d) contacting the polycation regions with a suspension comprising single cells, whereby a single cell is attached to each polycation region.

In some embodiments, the polyanion layer includes printing ink. In some embodiments, the polyanion layer includes 3 to 5 layers of printing ink. In some embodiments, the printing ink is liquid ink or toner.

In preferred embodiments, the polycation regions include polydiallyldimethyl ammonium chloride (PDAC). In some embodiments, the patterned array includes polycation regions having a size of about 5 μm to about 40 μm. In other embodiments, the patterned array includes polycation regions having a size of about 10 μm to about 20 μm. In a typical embodiment, the patterned array includes polycation regions having a size that allows attachment of a single cell.

Yet another aspect of the invention is method of predicting the efficacy of an anticancer treatment in a subject, the method comprising: (a) providing a plurality of cells from a subject; (b) exposing the cells to an anticancer treatment; (c) contacting the cells with the single cell array precursor of claim 1; and (d) quantifying DNA damage in the cells.

In some embodiments, the anticancer treatment includes radiation therapy. In some embodiments, the anticancer treatment includes chemotherapy drug. In some embodiments, plurality of cells is synchronized to be in the same cell cycle stage prior to exposure to the anticancer treatment. In some embodiments, DNA damage is quantified by calculation of the nuclear diffusion factor. In some embodiments, DNA damage quantification is automated.

In certain embodiments, step (c) further includes embedding the cells in agarose gel. In certain embodiments, step (c) further includes staining the cells with a fluorescent DNA dye.

In some embodiments, the method further includes the step of imaging the cells. In some embodiments, cells are imaged using fluorescence microscopy. In some embodiments, cells are imaged using a mobile phone camera.

The invention also can be summarized with the following listing of embodiments.

1. A single-cell array precursor, comprising:

a paper substrate;
a polyanionic layer deposited on a side of the paper substrate; and
a patterned array of polycationic regions deposited on the polyanionic layer.

2. The precursor of embodiment 1, wherein the polyanionic layer comprises printing ink.

3. The precursor of embodiment 1 or 2, wherein the polyanionic layer comprises 3 to 5 layers of printing ink.

4. The precursor of any of the previous embodiments, wherein the printing ink is liquid ink or toner.

5. The precursor of any of the previous embodiments, wherein the polycationic regions comprise polydiallyldimethyl ammonium chloride.

6. The precursor of any of the previous embodiments, wherein the patterned array comprises polycationic regions having a size of about 5 μm to about

40 μm.

7. The precursor of embodiment 6, wherein the patterned array comprises polycationic regions having a size of about 10 μm to about 20 μm.

8. The precursor of any of the previous embodiments, wherein the patterned array comprises a plurality of ordered polycationic regions, each having a size that allows attachment of only a single cell.

9. A single-cell array comprising the precursor of any of the previous embodiments and a plurality of cells individually attached to said polycationic regions.

10. The single cell array of embodiment 9, wherein each polycationic region is bound to at most a single cell.

11. The single cell array of embodiment 9 or 10, further comprising an agarose layer in which the cells are embedded.

12. A method of quantifying DNA damage in a plurality of individual cells, the method comprising:

(a) contacting the single-cell array precursor of any of embodiments 1-8 with said plurality of individual cells, whereby individual cells are attached to polycationic regions of said precursor, thereby forming a single-cell array;
(b) embedding the attached cells in an agarose layer covering the single-cell array;
(c) treating the cells with an alkaline solution to release their DNA into the agarose layer;
(d) imaging the DNA released from each cell in the agarose layer; and
(e) quantifying the released DNA for each cell.

13. The method of embodiment 12, wherein each polycationic region is bound to at most a single cell.

14. The method of embodiment 12 or 13, wherein DNA damage is quantified in step (e) by calculation of a nuclear diffusion factor.

15. The method of embodiment 14, wherein DNA damage quantification is automated.

16. The method of any of embodiments 12-15, wherein the cells are exposed to a DNA-damaging agent prior to or during step (a).

17. The method of any of embodiments 12-16, wherein step (c) further comprises exposing cells to a DNA-damaging agent.

18. The method of any of embodiments 12-17, further comprising the step of staining the cells with a fluorescent DNA dye.

19. The method of any of embodiments 12-18, wherein cells are imaged in step (d) using fluorescence microscopy.

20. The method of any of embodiments 12-19, wherein cells are imaged in step (d) using a mobile phone camera.

21. A method of making the single cell array precursor of any of embodiments 1-8, the method comprising:

(a) providing a paper substrate, a polyanionic material, and a polycationic material;

(b) coating the paper with the polyanionic material to form one or more polyanionic layers on a side of the paper substrate; and

(c) coating regions of the polyanionic layer with the polycationic material to form an array of polycationic regions, the regions sized to allow attachment of a single cell to each region.

22. The method of embodiment 21, wherein step (b) comprises the use of an inkjet printer to print the polyanionic material onto a surface of the paper substrate.

23. The method of embodiment 22, wherein step (b) comprises printing 3 to 5 layers of printing ink onto the surface of the paper substrate.

24. The method of any of embodiments 21-23, wherein step (c) comprises a microimprinting process.

25. The method of any of embodiments 21-24, wherein the polycationic material comprises polydiallyldimethyl ammonium chloride.

26. The method of any of embodiments 21-25, wherein the polycationic regions formed in step (c) have a size of about 5 $\mu$m to about 40 $\mu$m.

27. The method of embodiment 26, wherein the polycationic regions formed in step (c) have a size of about 10 $\mu$m to about 20 $\mu$m.

28. A method of making a single cell array, the method comprising:

(a) providing the single cell array precursor of any of embodiments 1-8 and a plurality of single cells;

(b) contacting the polycationic regions of the single cell array precursor with a suspension comprising the plurality of single cells, whereby single cells from the suspension become attached to the polycation regions.

29. A method of predicting the efficacy of an anticancer treatment in a subject, the method comprising:

(a) providing the single cell array precursor of any of embodiments 1-8 and a plurality of cells from a subject;

(b) exposing the cells to an anticancer treatment;

(c) contacting the cells with the single cell array precursor; and

(d) quantifying DNA damage in the cells.

30. The method of embodiment 29, wherein the anticancer treatment comprises radiation therapy.

31. The method of embodiment 29 or 30, wherein the anticancer treatment comprises a chemotherapy drug.

32. The method of any of embodiments 29-31, wherein the plurality of cells is synchronized to be in the same cell cycle stage prior to exposure to the anticancer treatment.

33. The method of any of embodiments 29-32, wherein DNA damage is quantified by the method of embodiment 12.

34. The method of any of embodiments 29-33, wherein DNA damage quantification is automated.

35. The method of any of embodiments 29-34, wherein step (c) further comprises embedding the cells in agarose gel attached to the single cell array precursor.

36. The method of any of embodiments 29-35, wherein step (c) further comprises staining the cells with a fluorescent DNA dye.

37. The method of any of embodiments 29-36, further comprising the step of imaging the cells.

38. The method of any of embodiments 29-37, wherein cells are imaged using fluorescence microscopy.

39. The method of any of embodiments 29-38, wherein cells are imaged using a mobile phone camera.

BRIEF DESCRIPTION OF THE DRAWINGS

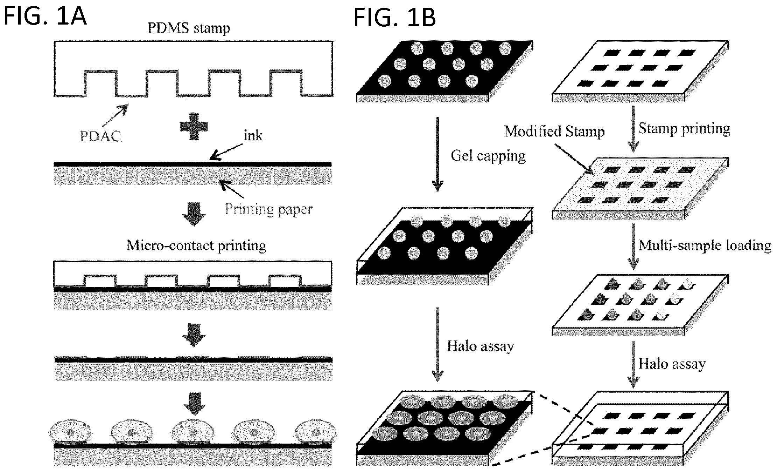

FIG. 1A shows a schematic representation of an embodiment of a method of forming a single cell array on ink-covered paper using soft-lithography. FIG. 1B shows a schematic representation of an embodiment of a DNA damage assay of invention.

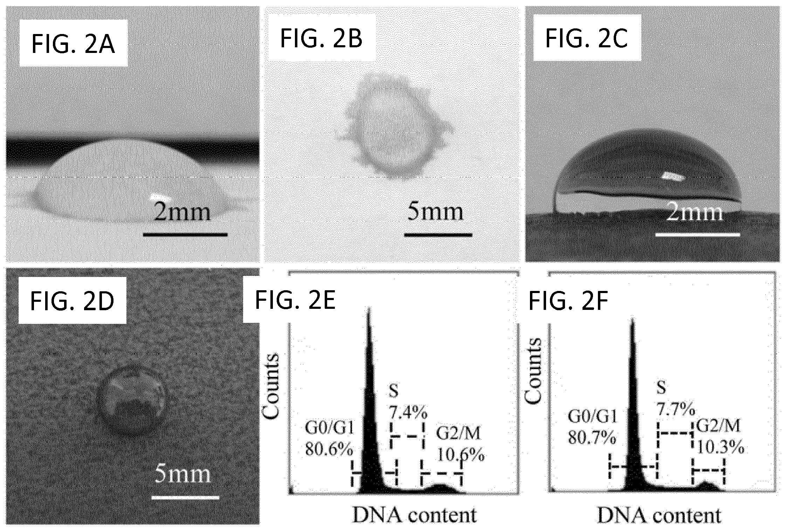

FIG. 2A shows the optical image of a drop of deionized water just after deposition onto bare paper. FIG. 2B shows a water drop on bare paper after 30 min. FIG. 2C shows a water drop deposited onto ink-covered paper. FIG. 2D shows a water drop on ink-covered paper after 30 min. FIGS. 2E-F show the assessment of DNA content of MCF7 cells detached from a petri dish (FIG. 2E) and ink-covered paper (FIG. 2F).

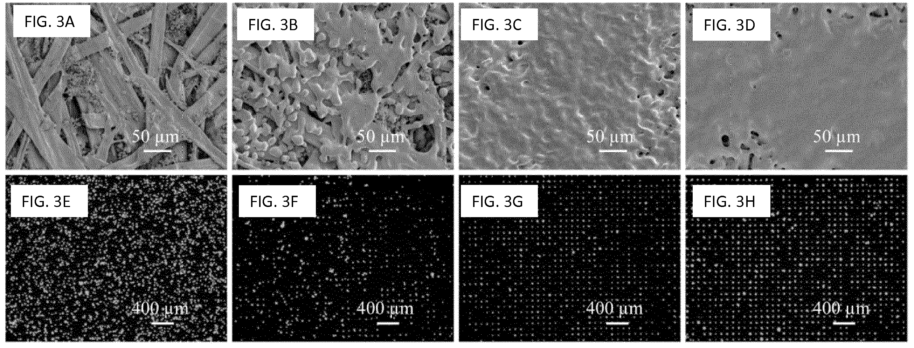

FIGS. 3A-D show scanning electron microscopy (SEM) images of bare paper (FIG. 3A), paper with one layer of ink (FIG. 3B), paper with three layers of ink (FIG. 3C), and paper with five layers of ink (FIG. 3D). FIGS 3E-3H show fluorescence micrographs of single cell arrays (stained using a Live/Dead assay) on paper without ink (FIG. 3E), with one layer of ink (FIG. 3F), with three layers of ink (FIG. 3G), and with five layers of ink (FIG. 3H).

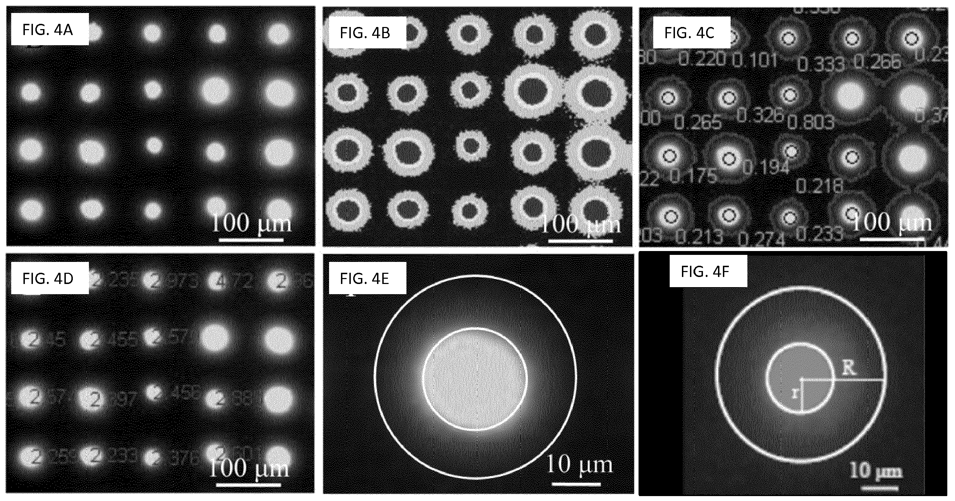

FIG. 4A shows grayscale cell images of a halo assay. FIG. 4B shows the identification of halos and nuclei in a defined array. FIG. 4C shows the identification of overlapped cells and halos. FIG. 4D shows calculated NDF values for each cell in the array. FIG. 4E shows an enlarged halo and nucleus image. FIG. 4F shows an enlarged cell with the halo (R) and nuclear (r) radii labeled.

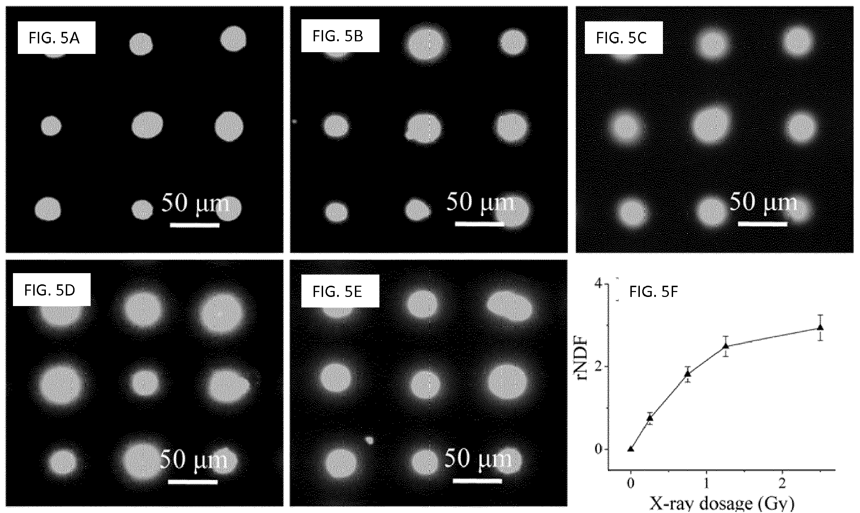

FIGS. 5A-E show X-ray induced DNA damage in MCF7 cells deposited in a single cell array on ink-covered paper. Fluorescence images of arrayed cells treated with doses of 0 (FIG. 5A), 0.25 (FIG. 5B), 0.75 (FIG. 5C), 1.25 (FIG. 5D) and 2.5 (FIG. 5E) Gy X-ray radiation. FIG. 5F shows the NDF values of X-ray radiation-induced DNA damage in the MCF7 cells of FIGS. 5A-5E.

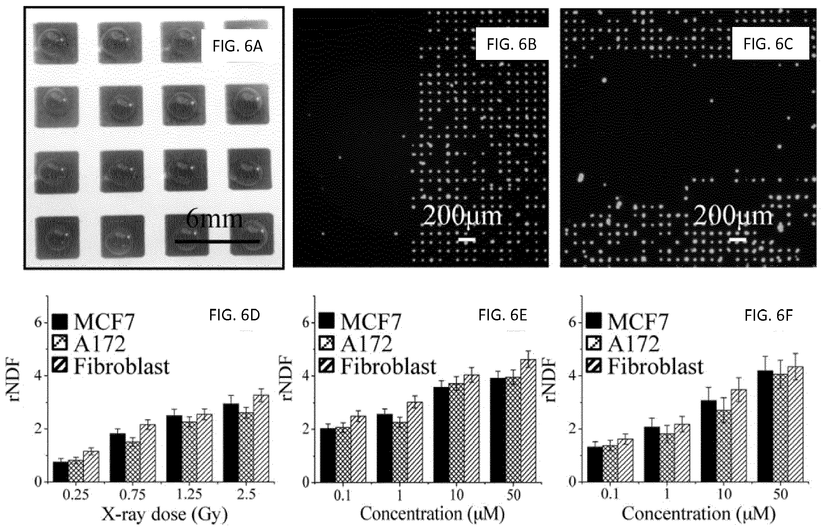

FIG. 6A shows an optical image of multiple samples loaded on ink-covered paper. FIG. 6B shows fluorescence images of the edge of a sample, and FIG. 6C shows the gap profile between two samples. FIGS. 6D-F show the dose dependence of NDF values indicating DNA damage in three cell lines (MCF7, A172 and fibroblasts) induced by X-ray radiation (FIG. 6D), doxorubicin hydrochloride (FIG. 6E) and CPT-11 (FIG. 6F).

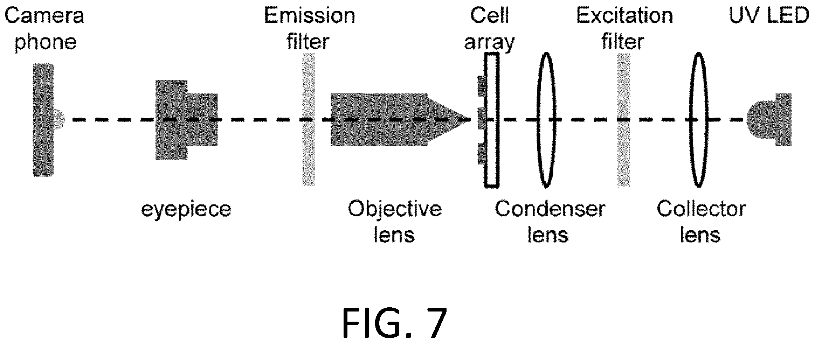

FIG. 7 shows a schematic representation of an embodiment of a system for fluorescence imaging of a cell array using a mobile phone camera.

## DETAILED DESCRIPTION OF THE INVENTION

The invention provides a low cost, high-throughput single-cell array to detect and quantify different types of DNA damage. The platform allows testing of multiple anti-cancer drugs and radiation dose conditions for a particular patient at point-of-care without expensive equipment or highly trained personnel. The platform can be further combined with widely-distributed mobile phone technology to allow image collection and data processing at clinics or in low-resource environments. This approach can assist in making cancer treatment personalized and more effective.

One aspect of the invention is a paper-based, single-cell array DNA damage assay. The assay is based on a classical halo assay combined with microfabrication techniques. Such paper-based single cell arrays can be used for essentially any single cell array-based assay, and particularly those that use fluorescence detection.

In an exemplary embodiment, a single cell array precursor is prepared by printing multiple layers of ink on paper, which is subsequently patterned with polyelectrolytes to create a series of polycationic features on the anionic substrate (i.e., ink-covered paper). The dimensions of the features are chosen to allow attachment of a single cell (FIG. 1A). The precursor is then contacted with cells that have been exposed to a DNA damaging agent, thus creating a single-cell array. The cells are subsequently embedded in agarose, lysed and stained with a fluorescent DNA dye (FIG. 1 B). After staining, characteristic halos form around cells, and the level of DNA damage can be quantified in a population of cells by determining the sizes of halos with an image processing program. Quantification can be fully automated, further facilitating the application of the technology at point-of-care or other low-resource environments.

Ordinary printing paper and liquid printing ink or toner can be employed. Alternatively, any charged polymeric material (preferably polyanionic, but can also be polycationic) can be used to coat a paper substrate, or a substrate made of other material such as an uncharged or charged polymer or silicon. The inventors have discovered that the negatively-charged ink from an inkjet printer can be used conveniently to coat paper and both block cell binding (since cells are also negatively charged) and also to block or obscure the background fluorescence of the paper. After depositing a layer of ink, or another negatively charged substance, such as a polyanion, a polycation is then deposited onto the negatively charged (e.g., inked) paper at spots where single cell attachment is desired, preferably using a soft lithography method. The array can be used for screening cells for their responses to drugs, which can be especially useful for testing anti-tumor drugs because of the heterogeneity of tumor cells within a patient.

In an embodiment, any substrate that can be printed on with regular printing ink can be used. Preferably, the substrate is cellulose-based printing paper. Regular liquid ink or toner printed on paper can effectively block fluorescence of paper materials, provide high affinity to charged polyelectrolytes, and prevent penetration of water into paper. The paper arrays are disposable and better suited for personalized medicine than silicon-based arrays.

Paper (including membrane) based materials have been utilized for biochemical analyses, including dipstick assays, lateral flow assays and microfluidic analytical devices, and can also be used as a substrate for a single cell array of the present invention. There are two major types of paper materials for point-of-care uses: one is cellulose fiber based materials such as filter paper and chromatography paper for dipsticks and microfluidic device, and the other is nitrocellulose membrane that is the key material for lateral flow assays. The porosity, surface chemistry and optical property of paper are critical for biochemical analysis. Surface chemistry can affect molecule or particle immobilization, non-specific adsorption and color expression. Both surface chemistry and porosity affect the wetting behavior of paper materials. The optical properties of paper affect accuracy of colorimetric and fluorescent readouts, as many commercial paper materials contain fluorescent molecules as brightening agents that produce high intensity fluorescence background.

Although paper-based approaches are affordable, user-friendly, rapid, robust and scalable, current paper-based diagnoses are limited to polymerase chain reaction and enzyme linked immunosorbent assay. Up to now, paper-based assays have not achieved widespread use for several reasons: (1) paper materials have a high fluorescence background, which prevents their use in fluorescence-based DNA damage assays; (2) the porous structure of paper materials has low affinity for cells, preventing their attachment and observation; and (3) there previously was no reliable and accurate way to detect DNA damage of cells attached on paper.

Normal printing paper is hydrophilic with a water contact angle of 40° as shown in FIG. 2A, where water spreading can be observed after several minutes (FIG. 2B). However, paper covered with 5 layers of ink shows hydrophobic properties, with a contact angle of 90° due to the presence of hydrophobic materials (i.e., wax and resin) added in ink (FIG. 2C). The hydrophobic ink prevents water from spreading on and penetrating into the paper. Water drops remained on the ink-covered paper even after 30 minutes (FIG. 2D).

Paper layered with ink has less surface roughness than uncoated paper. FIGS. 3A-D show scanning electron microscope (SEM) images of bare paper (FIG. 3A), and paper covered with one (FIG. 3B), three (FIG. 3C), and five (FIG. 3D) layers of ink. As the number of ink layers increases, the paper surface becomes smoother, and eventually individual fibers in the paper cannot be observed. The quality of single cell arrays is determined in part by surface roughness (FIGS. 3E-H). As the paper surface became smoother, more cells were attached, and the cell array became more ordered. In some embodiments, paper is covered with 1 or 2 layers of ink, or at least 3 layers of ink. Five layers of ink can provide a high quality substrate for single cell array formation. In some embodiments, paper is covered is covered with at least 4 or at least 5 layers of ink, for example, 5, 6, 7, 8, 9 or 10 layers of ink. Preferably the paper surface is hydrophobic, having a contact angle of at least about 50, at least about 60, at least about 70, at least about 80, at least about 90, at least about 100, or at least about 110 degrees.

Methods of fabrication

Soft lithography can be used to produce micropatterns for cell attachment (FIG. 1A). Such patterns can be, for example, a rectangular array having regular rows and columns at right angles, or any desired regular or irregular pattern. Low-cost and rapid cell patterning can be obtained by using soft lithography, or can be prepared using laser lithography. A solid master template with appropriate feature size can be generated on a template substrate using photolithography. The template substrate can be constructed of silicon, glass or a polymeric material, for example. In some embodiments, the template is an elastic material that can be cast or spun on a master template, polymerized, and then removed by pulling it away from the template. A suitable material is phenyldimethylsiloxane (PDMS). A PDMS stamp can be prepared by casting PDMS pre-polymer and curing agent against solid masters generated using photolithography. The stamp can have any dimension, geometry, or features as desired by the user. Preferably, the stamp includes raised features such as microposts, with sizes ranging from about 1 $\mu$m to about 100 $\mu$m; the feature sizes are selected to approximate the diameter of cells to be adhered in the single cell array, so that at most one cell can be attached to each raised feature. Even more preferably, the stamp includes microposts with sizes ranging from about 5 $\mu$m to about 40 $\mu$m, such as 10 $\mu$m, for the attachment of mammalian cells, for example.

The PDMS stamps can then have their surface modified to display positively-charged regions at the desired cell attachment features. The stamps can be modified, for example, by immersion in a cationic substance, such as polydiallyldimethyl ammonium chloride (PDAC), for example.

In some embodiments, a PDAC-modified PDMS stamp contacts the ink-covered paper, and a slight pressure is applied on the stamp, after which the stamp is peeled off from the paper. After removing the PDMS stamp, the PDAC layer is transferred onto the ink-covered paper due to electrostatic attraction between the negatively-charged ink and the positively-charged PDAC, thus creating polycationic regions. The polycationic regions, or features, act as attachment sites for cells. Cell attachment is easily achieved through interaction of positively charged islands made by the elastic stamp and the negatively charged cell membranes.

Single cell patterning requires adhesion area with size comparable to an individual cell. Since tumor cells of different origins may have different sizes, it is useful to employ the optimal size of adhesion area for the desired cell type. Optimal size for each cell line is found by considering the probabilities of zero, single and multiple cells on one feature. Feature size in the patterned array can be varied to maximize the probability of single cell attachment. The attachment efficiency can be derived by counting the number of cells attached on each feature. The optimized size for a selected cell line can be used to design a photolithography mask for that cell line.

In some embodiments, the patterned array has series of features with sizes from about 1 $\mu$m to about 100 $\mu$m. In some embodiments, the patterned array has adhesion areas with sizes ranging from about 1 $\mu$m to about 100 $\mu$m. In some embodiments, the adhesion areas of the patterned array comprise polycation regions having a size of about 1 $\mu$m to about 100 $\mu$m. In preferred embodiments, the adhesion areas of the patterned array comprises polycation regions having a size of about 5 $\mu$m to about 40 $\mu$m. In even more preferred embodiments, the adhesion areas of the patterned array comprises polycation regions having a size of about 10 $\mu$m to about 20 $\mu$m.

The spacing between adjacent features determines surface density of patterned cells, and the total number of cells that can be patterned at a given area. Therefore, smaller spacing gives higher density, but may increase the probability of multiple cell attachment, which is preferably avoided in any single-cell assay. The spacing between features can be chosen as a function of feature size: in some embodiments, the spacing is half the size of the features or has the same size as the features.

In preferred embodiments, the spacing is larger than the feature size. In some embodiments, feature size is from about 5 μm to about 40 μm, and the spacing between features is from about 50 μm to about 400 μm. In preferred embodiments, the spacing between features is about 100 μm. A chip containing features with a spacing of 100 μm can avoid possible overlapping of halos from adjacent cells while maintaining good cell density ($10^5$ cells on a 15 mm x 15 mm chip).

Applications

DNA damage is an alteration in the chemical structure of DNA, such as single-and double-strand breaks, oxidation, alkylation or hydrolysis of bases, mismatch of bases, and DNA crosslinking. These lesions can block transcription and replication, leading to cell senescence and death. DNA damage is the underlying basis of most cancer therapies such as chemotherapy and radiation therapy. However, individuals greatly vary in their response to DNA damaging agents and in their ability to repair each type of DNA lesion. In the context of personalized medicine, evaluating drug- or radiation- induced DNA damage in extracted cancer cells allows doctor to identify the best available treatment for each patient before prescription, thus greatly enhancing treatment efficacy and minimizing adverse effect. Moreover, a significant challenge in cancer therapy is the development of drug resistance by tumors, and there is evidence that DNA repair ability is linked to tumor resistance in chemotherapy.

In one embodiment, a tumor tissue sample or blood sample is obtained from a cancer patient. The sample can be processed to produce isolated cells, such as by trypsinization. The cells can then be suspended in solution and subjected to treatment with a genotoxic drug or radiation exposure for the desired period of time. After treatment, cells can be contacted with the single-cell array precursor, thus forming a single cell array, after which the cells can be encapsulated in agarose gel. Damaged DNAs will unwind and diffuse inside the gel with diffusion coefficients inversely proportional to DNA fragment sizes. After fluorescent staining of DNA, the cells are imaged using fluorescence microscopy, where fluorescence signals are proportional to the amount of DNA. The level of DNA damage is quantified by using a relative nuclear diffusion factor (rNDF) derived from the surface areas of the nucleus and halo.

The agarose provides an inter-connected network for relaxed DNA fragments to diffuse, with diffusion rates being dependent on DNA chain lengths. High concentrations of agarose facilitates separation of short DNAs, while low agarose concentrations allow resolution of large DNAs. Agarose gels (both high melting point and low melting point) in different concentrations can be used for DNA diffusion with the single-cell array of invention. The gel pore size can be determined with absorption spectroscopy in the 700-800 nm range. Preferably, the agarose is low melting point agarose. Preferably, the concentration of agarose is about 1%.

In one embodiment, after being encapsulated in agarose, cells are subjected to alkaline lysis in order to allow histone dissociation from DNA chains and consequent release of damaged DNA. The alkaline lysis can be conducted as a regular fast alkaline halo assay. For example, the array can be immersed in 0.3 M NaOH for 15 min, and rinsed with water to remove the NaOH.

The present invention allows for the investigation of a variety of types of DNA damage, including single- and double-strand breaks, and DNA crosslinking. Some anti-cancer drugs such as cisplatin and mitomycin C (MMC) cause DNA crosslinking, but no breaks. Since crosslinked DNA migrates less than that of control cells, in some embodiments, drug-treated and control cells can be subjected to a second genotoxic agent (ionizing radiation or methyl methanesulfonate) and the extent of DNA diffusion (i.e., rNDF) in the presence and absence of a reference genotoxic agent can be compared. Double strand breaks (DSBs) and single strand breaks (SSBs) of DNAs can be differentiated by using the invention at different conditions. For example, under certain non-denaturing conditions, DSBs will be detected without interference from SSBs; while under certain denaturing conditions, both DSBs and SSBs will be detected simultaneously. In some embodiments, the non-denaturing condition is a solution containing 0.15 M NaOH, 100 mM $NaH_2PO_4$, 1 mM EDTA free acid, 1% Triton X100, at pH 10.1. In some embodiments, the denaturing condition is a solution containing 0.3 mM NaOH, at pH 13.

The invention can also be used to test DNA response to damage, instead of whole cell response to DNA damage. Acellular assays can be performed in which untreated cells are patterned on a substrate to form a cell array, embedded in an agarose gel, and lysed. The liberated DNAs will then be subjected to drug or radiation for a certain period of time to evaluate DNA damage. Considering that DNAs - and not cells - are exposed, this acellular assay will reflect the ability of drug or radiation to cause DNA damage independent of cytotoxicity.

The invention can also be used in place of other common cytogenetic testing procedures, such as fluorescence in situ hybridization (FISH). A common application of FISH is hybridization performed in cells of patients with breast cancer for the purpose of treatment decisions. The antibody drug trastuzumab, for example, can only be administered to breast cancer patients who carry excess copies of the HER2 (human epidermal growth factor receptor 2) gene. FISH is carried out in order to detect the presence of an altered number of copies of the gene, and thus inform on the appropriate course of treatment. However, FISH is a relatively expensive procedure that often gives inaccurate results. Moreover, it has been reported that even women who are negative for HER2 amplification may benefit from treatment with trastuzumab (Carlson, Biotechnol Healthc. 2008 Sep-Oct; 5(3): 23-27). In summary, it would be preferable to evaluate a patient's response to trastuzumab directly, instead of relying on inconsistent proxies for drug efficacy. The invention can thus be used to substitute for FISH in clinical settings, by providing information on the actual efficacy of trastuzumab against a patient's tumor cells. Patient's cells can be incubated with trastuzumab and, optionally, other chemotherapeutic drugs, and processed with the method of invention to assess DNA damage. Further, the repair abilities of cells can be studied by washing away trastuzumab, and putting cells back into incubation at 37°C in 5% $CO_2$. After the desired incubation time, cells can be processed with the invention to quantify the amount of damaged DNA. A single-cell array of the invention can provide useful evidence for treatment decisions, since ~50% of HER2 positive patients do not respond to trastuzumab therapy.

Yet another application of the invention is to allow probing of protein expression with immunoblotting. Some anti-cancer drugs are effective inhibitors of DNA topoisomerase I and II (topo1 and topo2). They can stabilize transient cleavable complexes (topo1-DNA and topo2-DNA) and further induce secondary lesions that consist of irreversible DSBs and SSBs, respectively. In addition, an early event upon DSB induction is serine phosphorylation of carboxy-terminal tail of histone variant H2AX to γH2AX. So, identification of topoisomerase complexes with DNA and γH2AX at single cell level can provide mechanistic confirmation on drug- or radiation-DNA interaction. In some embodiments, cells are treated with drugs targeting one or both topoisomerases, followed by immunoblotting.

Image Acquisition and Analysis

After being processed, such as for halo analysis, the cells in a single-cell array can be imaged. For imaging (e.g., for halo analysis), cells can be stained with any fluorescent DNA-binding dye, such as ethidium bromide or SYBR SAFE. Characteristic halos form around cells after staining DNA with a fluorescent dye. The level of DNA damage can then be quantified by determining the sizes of halos and nuclei.

Imaging can be performed using any suitable means, including a conventional fluorescence microscope or in a simple imaging microscopy system including a mobile phone camera as the imaging and image processing device. FIG. 7 shows an exemplary embodiment in which fluorescence imaging of a cell array is performed with a phone camera. A UV light emitting device (LED) and filters can be aligned in trans-illumination geometry, and mounted on an optical rail together with the phone and optical components. A microscope eyepiece can be placed between the camera and microscope objective. An excitation filter can be placed between the collector lens and condenser lenses, and an emission interference filter can be placed close to the back focal plane of objective lens. In some embodiments, the camera is used as a high-quality microscope, and the phone performs image collection, data analysis, and treatment recommenations.

One key advantage of the present invention is the ease and reliability of DNA damage quantification. The quantification can be fully automated, requiring no user intervention and no special equipment or complex software owing to clear boundary, symmetric shapes of halos and nuclei, and non-overlapping nature of adjacent cells/halos. Any suitable image processing program can be employed to quantify DNA damage. DNA damage measurements are calculated based on the halos and nuclei labeled for each cell. Nuclear diffusion factor (NDF) is a measure of the relative surface area of the entire cell, including halo and nucleus, to the nucleus alone. Computationally, NDF is found by measuring the area of the halo, $A_h$, and the area of the nucleus, $A_n$, and relating them with the following formula:

$$NDF = \frac{A_h + A_n}{A_n}$$

An NDF from a control experiment, i.e., where no DNA damage occurs, is obtained. A relative NDF value (rNDF) is obtained by subtracting the NDF value of treated cells from the NDF of an untreated control. DNA damage measurement is shown for each cell (FIG. 4D). FIG. 4E shows an enlarged cell for better visualization of nucleus and halo. FIG. 4F shows an enlarged cell with labels indicating the radii of halo (R) and nucleus (r).

EXAMPLES

Example 1. Materials and Methods.

*PDAC-modified PDMS stamps.* Polydimethylsiloxane (PDMS Sylgard 184) stamps were prepared by casting PDMS pre-polymer and curing agent against solid masters generated using photolithography. The unmodified PDMS stamp had microposts with diameter of 10 μm. The unmodified PDMS stamp was immersed in polydiallyldimethyl ammonium chloride (PDAC) (100,000-200,000 Da) solution for 15 min at room temperature, rinsed by deionized water, and dried in a gentle nitrogen stream.

*Cells.* Fibroblast cells, human glioblastoma cells (A172) and breast cancer cells (MCF7) were cultured in standard conditions (5% $CO_2$ in air at 37°C) in RPMI-1640 medium supplemented with 10% (v/v) fetal bovine serum and 1% (v/v) penicillin/streptomycin. For obtaining individual cells, cells were trypsinized.

Example 2. Production of Single-Cell Array Precursor.

Bare paper was covered with ink by printing 1 to 5 dark layers on normal printing paper using a Cannon MF4890dw printer. Paper with different layers of ink was found to have different surface roughness. FIGS. 3A-D show scanning electron microscope (SEM) images of bare paper, and paper covered with one, three and five layers of ink. As layers of ink increase, the paper surface became smoother, and fibers in the paper could not be observed clearly. The PDAC-modified PDMS stamp containing 10 $\mu$m-microposts was brought into contact with the ink covered paper, and a slight pressure was applied on the stamp for 15 s to ensure conformal contact between the stamp and ink-covered paper, after which the stamp was peeled off from the paper. After removing the PDMS stamp, the PDAC layer was transferred on the ink-covered paper due to electrostatic attraction between the negatively-charged ink and the positively-charged PDAC.

Example 3. Formation of Single-Cell Array.

After exposure to anticancer treatment (radiation or chemotherapy drug), cells were seeded on the cell array precursor at a density of $1 \times 10^6$ cells/ml, forming ordered single-cell arrays (FIG. 1A). The quality of single cell arrays was primarily determined by surface roughness of the substrate (FIGS. 3E-H). As the paper surface became smoother, more cells were attached, and the array became more ordered. The attachment probability was derived as the ratio between adsorbed cells and the number of micro-patches. The probability of single cell array increased as the number of layers increased from 25 $\pm$ 3.1%, 70 $\pm$ 3.5%, to 87 $\pm$ 6.4% for 1, 3 and 5 layers of ink, respectively. Five layers of ink provided a high quality substrate for single cell array formation, making this method ideal for paper-based single-cell halo assay.

After incubation for 30 min, unattached cells were rinsed away by using PBS. In order to confirm that arrayed cells were still alive, cells were tested with Live/Dead viability/cytotoxicity assay, where dead cells are labeled as red, and living cells are labeled as green. Fluorescence images (FIGS. 3E-H) showed that all arrayed cells were alive (bright spots), and round after patterning on ink-covered paper for 1 hour.

After incubating cells on the paper for 30 min, unattached cells were rinsed away with PBS, followed by addition of 1.5 ml 1% low melting point (LGT)-agarose on paper. The substrate was kept at room temperature for 10 min to allow gel solidification. Then, cells on the paper were immersed in 0.3 M NaOH for 30 min at room temperature, and stained with diluted ($\times$10000) SYBR green I solution for 15 min. After removing unbound dye, cells were imaged using an Olympus IX81 fluorescent microscope and the acquired images were analyzed with in-house image analysis software to quantify DNA damage. Each data point was averaged from at least 50 individual cells.

Example 4. Image Processing.

An in-house MATLAB image processing software was developed for quantifying DNA damage. In order to isolate individual cells, images were thresholded using Otsu's method into 4 levels. The first level was considered background; the second and third levels were combined to label halo; and the fourth level was used to label the nucleus. All non-background levels were combined to form a binary image of a cell. The eccentricity of each cell object was measured on a scale of 0 to 1, where a score of 0 corresponded to a perfectly circular object and a score of 1 corresponded to a line. Cell objects with an eccentricity > 0.5 were removed from the image on the assumption that they contain overlapping cells (due to deposition of un-patterned cells). FIGS. 4A-F show the process of automatic analysis of the single-cell array using MATLAB software. The original image was transferred from a color image to a grayscale image (FIG. 4A). The image was thresholded into 4 levels (FIG. 4B), where red color (core) corresponds to cell nuclei, yellow and cyan colors to halos, and blue color to background. Cell objects were outlined and demonstrated next to an eccentricity measurement. Cell objects passing the selected criteria with a measure of 0.500 or less were marked with a black circle at their centers (FIG. 4C). DNA damage measurements were calculated based on the halos and nuclei labeled for each cell.

Nuclear diffusion factor (NDF) is a measure of the relative surface area of the entire cell, including halo and nucleus (R), to the nucleus alone (r) (FIG. 4F). Computationally, NDF is found by measuring the area of the halo, $A_h$, and the area of the nucleus, $A_n$, and relating them with the following formula:

$$NDF = \frac{A_h + A_n}{A_n}$$

An NDF from a control experiment (no DNA damage occurs) was obtained. A relative NDF value (rNDF) was obtained by subtracting the NDF of treated cells from the NDF of the untreated control. DNA damage measurement is shown for each cell in FIG. 4D, and an enlarged cell is shown in FIG. 4E. The results were derived from fully automated imaging and analysis with no user intervention and no special equipment or complex software owing to clear boundary, symmetric shapes of halos and nuclei, and non-overlapping nature of adjacent cells/halos.

Example 5. X-Ray Radiation-Induced DNA Damage on Paper.

The arrayed cells were exposed to X-ray radiation produced using a Mini-X X-ray tube from Amptek (Bedford, MA) with a silver anode operating at 40 kV and 100 $\mu$A. The tube was fitted with a brass collimator (with a 2 mm diameter pinhole) to focus X-rays onto the paper.

After radiation exposure, cells were contacted with the single-cell array precursor and embedded in 1% low-melting-point agarose. After gel solidification (10 min at room temperature), cells were stained with SYBR Green I dye. The dye can insert in the DNA double helix, and the fluorescence intensity is proportional to amount of DNA (as DNA is stained randomly with the dye). FIGS. 5A-E show the fluorescence images of cells exposed to different dose of X-ray (0, 0.25, 0.75, 1.25 and 2.5 Gy, respectively). The control cells showed no DNA diffusion from the nucleus. DNA was entirely localized within the nucleus and appeared as a bright fluorescent circle (FIG. 5A). As X-ray dose increased, the amount of damaged DNA also increased, and the nuclear area became dimmer and the halo became bigger (FIGS. 5B-E). FIG. 5F shows the rNDF values of MCF7 cells exposed to different doses of X-ray, where NDF values increased from 0.74 to 2.94 when X-ray dose increased from 0.25 to 2.5 Gy. FIG. 6D shows the NDF of cells after exposure to X-ray radiation. As X-ray doses increased from 0.25 to 2.5 Gy, rNDF values increased from $0.83 \pm 0.12$ to $2.61 \pm 0.21$ for MCF7 cells, $0.75 \pm 0.14$ to $2.95 \pm 0.31$ for A172 cells, and $1.17 \pm 0.13$ to $3.27 \pm 0.24$ for fibroblast cells, showing that higher X-ray dose causes more DNA damage.

## Example 6. High-Throughput DNA Damage Assay.

Multiple drugs can be tested simultaneously by loading drugs at different isolated areas of the single-cell array precursor. FIG. 6A shows an optical image of multiple samples loaded on different regions of the ink-covered surface, where each black region is a patterned array of PDAC islands. Each sample was loaded onto an ink-covered square to form a single-cell array. After embedding cell arrays inside an agarose gel, cells were treated with alkaline solution, stained, and washed to remove excess dye. FIGS. 6B-C show the fluorescent images of the sample edges, where ink-covered areas block cells well, and there is no cross contamination between two adjacent samples.

Two genotoxic drugs were used: doxorubicin hydrochloride (579.99 g/mol) and irinotecan hydrochloride (CPT-11, 623.14 g/mol). FIG. 6E shows the NDF values of cells after exposure to doxorubicin at different concentrations. As drug concentration increases from 0 to 50 $\mu$M, NDF of MCF7 cells increases from 0 to 3.95, that of A172 cells increases from 0 to 3.91, and that of fibroblast cells increases from 0 to 4.63. FIG. 6F shows rNDF values of cells after exposing to irinotecan at various concentration. As the concentration increased from 0 to 50 $\mu$M, NDF of MCF7 cells increased from 0 to 4.05, and that of A172 cells increased from 0 to 4.19, while that of fibroblast cells increased from 0 to 4.34. These results indicate that higher concentrations of chemotherapeutic drugs caused more DNA damage.

## Example 7. Effect of Cell Attachment to Single-Cell Array Precursor on Cell Cycle.

In order to exclude the possibility that surface attachment of cells on paper may change cell cycle, cells patterned on the ink-covered paper were detached, collected by centrifugation at 1200 rpm for 4 min at room temperature, and then suspended in 1 ml ice cold PBS buffer. Cell suspension was added drop-wisely to 9 ml of 70% ethanol and stored at 4°C to for 2 hr. Cells were collected from ethanol by centrifugation at 1200 rpm for 10 min at 4°C. The collected cells were stained with 500 $\mu$l propidium iodide (PI, 20 $\mu$g/ml) containing 0.1% Triton X-100 for 15 min at 37°C, and assessed with a BD Accuri C6 flow cytometer (BD Biosciences). The data were processed with OriginPro 8.5, and presented as the mean with a standard deviation. The statistical significance of results was determined by means of an analysis of variance using the SPSS software (SPSS 19.0, IBM, Armonk, NY). Comparisons between control group and treatment group were based on t-test. A result was considered statistically significant difference when $P \leq 0.05$. The final results represented the mean of at least three independent experiments. Cells detached from petri dish are used as a control. DNA content within cells is taken as a marker of cellular maturity. FIGS. 2E-F show that DNA content in each cycle was similar for cells patterned on the ink-covered paper and those on the petri dish.

## Example 8. Comparison of single-cell array with fluorescence in situ hybridization.

A single-cell array is tailored to carry out FISH on cells from breast cancer patients (hybridization with HER2 gene), as well as to monitor the efficacy of trastuzumab-doxorubicin treatment concurrently. Two different cell lines that have different levels of HER2 expression are used: SKBR-3 cell lines expressing high level of HER2 antigen, and MCF-7 cell lines expressing low level of HER2 antigen. First, FISH will be performed on cell arrays to determine HER2 expressions in both cell lines. HER2 amplification will be examined after forming single cell arrays using PathVysion HER2 FISH Probe Kit, which contains dual color probes for HER2 gene and centromere of chromosome 17 (CEP17), respectively. CEP 17 will be used as an internal control to account for aneusomy of chromosome 17. From this FISH test, both absolute HER2 copy number and HER2/CEP17 ratio are derived from fluorescence images, where red and green dots reflect HER2 genes and CEP17, respectively. 4',6-diamidino-2-phenylindole (DAPI) is used to stain nuclear DNA as a blue background. Second, both cells are incubated with a solution of 10 $\mu$g/ml of trastuzumab and 10 $\mu$g/ml doxorubicin, and processed with the invention to quantify DNA damages with rNDF values. Cell lines with higher HER2 level have higher rNDF values. Further, the repair abilities of both cells can be confirmed by washing away trastuzumab, and putting cells back into incubation at 37°C in 5% $CO_2$. After varied incubation times, cells are taken out and processed with an assay according to the invention to quantify the amount of damaged DNA. The concurrent FISH test and drug efficacy evaluation with the single-cell array provides useful evidence for cancer therapy decision making.

Example 9. Use of single-cell array with immunoblotting

Identification of topoisomerase complexes with DNA and histones at single-cell level can provide mechanistic confirmation on drug- or radiation-DNA interaction. Three colorectal cancer cell lines, HCT-116 (normal topo1/topo2 expression), HCT-116-siRNA-topo1 (low topo1 expression) and HCT-116-siRNA-topo2 (low topo2 expression), are treated with two drugs (one targets topo1 and the other targets topo2), followed by immunoblotting with the array of invention. First, cells are treated with CPT-11 (topo1 drug), and processed for use with the invention using SYBR SAFE dye to reflect the extent of DNA damage. Halo size is the smallest in HCT-116-siRNA-topo1 cells compared with HCT-116 and HCT-116-siRNA-topo2 cells. In a second experiment, CPT-11 treated cells are subjected to an alkaline solution to allow damaged DNA diffusion (without fluorescence staining), and then incubated with three primary antibodies (topo1, topo2 and γH2AX) for 10 min. After washing to remove excess antibodies, three fluorescent secondary antibodies against topo1, topo2 and γ-H2AX are added. After washing away excess antibodies, the three proteins are detected with fluorescence imaging. Only topo1 is found in halos (topo1 enzymes are trapped in damaged DNAs in halo); topo2, γ-H2AX and some topo1 is found in the nucleus, while there is much less topo1 in the halo of HCT-116-siRNA-topo1 cell. Parallel studies with VP-16, which targets topo2 enzyme is performed. Only topo2 enzymes are trapped in damaged DNAs and diffused in halos; topo1, γ-H2AX and some topo2, is located inside the nucleus, while there is a much lower level of topo2 in halo of HCT-116-siRNA-topo2 cell. Similar experiments are performed to test X-ray irradiated cells. The results are compared to provide information on drug actions.

Example 10. Identification of lesion-specific DNA damage.

Use of enzymatic digestion or FISH along with the single-cell array will allow identification of broad classes of DNA damages (cross-linking, alkylation, oxidation), as well as gene-specific DNA damage. An enzymatic digestion step with a lesion-specific enzyme will be used to convert various DNA damages into strand breaks. The invention will be used to detect oxidized bases: after drug or radiation treatment, cells will be embedded in a gel; formamidopyrimidine-DNA glycosylase will be added to cause DNA strand breaks at oxidized purine sites; or endonucleases III will be added to cause strand breaks at oxidized pyrimidine sites; cells will be subjected to the method of invention to derive rNDFs of enzyme-treated cells. A control experiment without enzyme digestion will be done to derive rNDFs, which will be subtracted from the ones from enzyme treated cells. In addition, the array will be used to identify alkylation damage using 3-methyladenine DNA glycosylase II (Alk A) with the same approach.

In order to test gene-specific DNA damage, the array will be combined with FISH to assess the therapeutic effects of drugs such as bleomycin (BLM) and mitomycin C (MMC) on telomere shortening. It is known telomere erosion or loss is an early sign in DNA damage-induced apoptosis. Telomere-specific peptide nucleic acid (PNA) hybridization probes will be used to detect DNA fragmentation caused by BLM and MMC in human cancer cells. Cells will be treated for 1 h with BLM or for 2 h with MMC at 37°C at certain concentrations. The drug-treated cells will be processed with single-cell array and FISH assays at the same time. Telomere-specific PNA probes (Telomere PNA FISH Kit/Cy3) will be used to detect telomeric DNA sequences in the damaged DNA of cells. The cells will be stained with SYBR Green to quantify DNA damage. The number of telomere signals, the localization of signals (either inside halo or core), and rNDF values will be recorded for each cell. The comparative levels of telomeric DNA damage and global DNA damage will be assessed from the combined use of the invention and FISH assays.

Example 11. Drug testing with patterned lymphocyte cells.

Lymphocytes in blood samples (cancer patients and health controls) will be used to test drug response using the invention. In order to prepare peripheral white blood cells (buffy coat), 10 ml of blood will be drawn, put in a heparinized tube, and diluted with 10 ml DMEM (Dulbecco's modified eagle medium). The diluted blood will be carefully layered on a density gradient Ficoll-Hypaque. After centrifugation, the lymphocyte band will be harvested without touching Ficoll using a sterile pipette tip, and washed twice with ice-cold DMEM medium by centrifugation at 1,200 rpm for 10 min. Lymphocytes will be suspended in a complete RPMI 1640 medium, and cell viability will be examined by trypan blue dye exclusion. A particular cell population (such as T and B cells) can be further isolated from a sample by passing over columns of antibody-coated, nylon-coated steel wool. The cells will be diluted 1:10 in RPMI 1640, and treated with CPT-11, followed by cell patterning and agarose embedding to quantify DNA damage with the array of invention. The repair abilities of lymphocytes will be determined by incubating drug treated cells for different intervals (0.5, 1, 3 and 24 hr), and quantified the DNA damage as rNDF value for each interval. The results will be compared for different types (B and T cells), and different batches of lymphocytes (different patients and healthy controls) to reveal inter- and intra-patient variability of DNA damage. Lymphocytes from cancer patients are expected have increased DNA damage and reduced DNA repair ability compared with healthy controls.

Example 12. Drug testing with patterned circulating tumor cells.

Circulating tumor cells (CTCs) released from a primary tumor into patient blood can be a predictive marker for treatments. The invention will be used test drug response of CTCs patterned on the substrate. De-identified human blood samples from breast cancer patients will be obtained from University of Massachusetts Memorial Hospital. Due to their low concentration (10 CTCs in 1 ml blood at early stage cancer, and 100-1000 CTCs in 1 ml blood in late stage cancer), CTCs in blood will first be enriched with immune-magnetic nanoparticles before drug treatment. It is postulated that magnetic nanoparticles will catch CTCs, but will not affect electrostatic attraction between CTCs and surface patterns (due to small size of nanoparticles). Briefly, super-paramagnetic iron oxide nanoparticles will be modified by APTES to have amine groups, and treated with 0.1 mM n-gamma-maleimidobutyryloxy succinimide ester (GMBS) in dimethyul sulfoxide (DMSO) for 30 min. These nanoparticles will be conjugated to streptavidin (10 $\mu$g/ml in PBS for 1 h). The modified nanoparticle will be grafted with anti-EpCAM (epithelial cell adhesion molecule) antibodies by dispersing in PBS containing 10 $\mu$g/ml anti-EpCAM antibody, 1% (w/v) bovine serum albumin (BSA), and 0.1% (w/v) sodium azide for 30 min. After washing to remove excess antibodies, the nanoparticles will be dispersed into blood for some time. A magnet will be used to collect magnetic nanoparticles and CTCs, which will allow CTCs to be enriched to a smaller volume (50 $\mu$l). The collected CTCs will be treated with an anti-cancer drug (CPT-11), and patterned onto a solid substrate to form single CTC array, which will be followed by gel capping, dye staining and DNA damage quantification.

Example 13. Drug testing with cells extracted from solid tumor tissues.

De-identified tumor tissue samples of colorectal cancer patients and healthy controls will be obtained with colonoscopy from University of Massachusetts Memorial Hospital. The tissue will be minced and enzymatically dissociated to form cell suspensions with isolated cells. Briefly, the tissue will be minced into 1 mm fragments, which will be exposed to digesting medium in a trypsinizing flask containing RPMI Medium 1640, 10% fetal calf serum, 0.1% DNAse Type I, and 0.14% collagenase type I. After incubation, fragments will be allowed to pass through a 70 to 200 micron filter to remove aggregates. The preparation will be done on ice. After removing supernatant by centrifugation, pellets will be washed with 0.9% NaCl, and resuspended in 0.9% NaCl. Cell viability will be checked by live/dead assay. Cells will be transferred into PBS solution, and exposed to two drugs (CPT-11 and 5-fluorouracil) at different concentrations, followed by processing with the invention to quantify DNA damage. DNA repair abilities will be determined by incubating drug treated cells for different intervals (0.5, 1, 3 and 24 hr), and quantified DNA damage as rNDF value for each interval. Differences in terms of chemosensitivity to drug will be derived from rNDFs values. The patient whose tissue produces a large halo will be considered sensitive to a particular drug, and the patient whose tissue produces a small halo is insensitive to the drug, and thus will not be recommended to take that drug.

Example 14. Radiation testing with cells extracted from solid tumor tissues.

Radiation therapy can damage DNAs of tumor and normal cells. Radiation conditions are chosen in such a way that normal cells repair damaged DNAs, while tumor cells cannot and will undergo apoptosis. It is assumed DNA damages at low X-ray energy (voltage 0-100 kV) and flux (current 10-100 $\mu$A) can be extrapolated to predict DNA damages at high energy X-ray generated from cobalt 60 source. In order to determine the ability of X-ray irradiated cells to repair sublethal DNA damage, cells will be irradiated with X-ray at certain dose. Some cells will be immediately patterned on a solid substrate, and embedded in an agarose gel for DNA damage quantification using the invention. The rest of cells will be incubated at 37°C in 5% $CO_2$ atmosphere for 1, 2, 4, 6, 8 and 12 h before they are processed with the invention to quantify DNA damages. In order to compare DNA damage quantified with the invention with the gold standard of measuring cell radiosensitivity (the ability of cell to form colony), the colony formation ability of X-ray irradiated cells will be determined by counting how many cells are able to form colony under in vitro culture condition with an optical microscope. X-ray energy and flux dependent DNA damages will be studied by irradiating cells with X-ray of different energy with 5 kV increment. The trend of DNA damage will then be extrapolated to predict cell response at high energy therapeutic conditions. In order to derive DNA damage-dose curve, the trend of DNA damage will be compared with the trend of cell survival (dose-rate curve) at the same dose level. By plotting dose on linear scale and DNA damage (surviving fraction) on logarithmic scale, the curves will be fitted to determine whether radiation damage follows single-target model, multi-target model or linear quadratics model. The effect of radiation dose fractionation will also be tested with the invention. Cells will be subjected to the similar irradiation-recovery-irradiation cycle as that used in radiation therapy. DNA damages after each operation will be quantified with the invention. In comparison, another batch of cells will be irradiated with single irradiation with the same total dose, and processed with the invention to quantify DNA damage. The cell survival rates from both irradiation formats will also be compared. Differences in terms of radiosensitivity will be derived from rNDFs values. The patient whose tissue produces a large halo at one radiation condition will be considered sensitive, and will be recommended to take that for treatment.

Example 15. Image collection and data processing with mobile phone

An iPhone with 8 megapixel CMOS camera will be used for fluorescence imaging, where a UV light emitting device (LED) and filters will be aligned in trans-illumination geometry, and mounted on an optical rail together with the phone and optical components (FIG. 7). A 20X wide field microscope eyepiece will be placed at 5.6 mm (focal length of camera) away from the camera, and 160 mm away from a microscope objective (60X 0.85 NA DIN Achromat). A 5° spot lens acting as a collector lens will be mounted on LED, and placed 11 cm away from a 25.4 mm focal length condenser lens. An excitation filter will be placed between the collector lens and condenser lenses. An emission interference filter will be placed close to the back focal plane of objective lens. Bright field images will be taken using camera's default setting when flash is disabled; and fluorescence images will be captured in night mode with flash disabled. A background image from an area with no fluorescent signal will be subtracted from a sample image. The images in JPEG (joint photo-graphic experts group) format will be split into red-green-blue layers, and green channel (fluorescence emission of SYBR Safe dye) will be retained. An imaging processing software based on iPhone system will be created using MATLAB software. The program will be able to resolve a halo and a nucleus based on fluorescent intensities, extract the halo and nucleus diameter and calculate a nuclear diffusion factor for each cell. Statistical analyses of data will be performed with Statistics Package for the Social Sciences software, version 17 (SPSS, Inc. Chicago). All values will be reported as means $\pm$ SD for all the experiments. A non-parametric, Mann-Whitney U-test will be used to compare values. Student's t-test will be used to check sample's variability. $p < 0.05$ will be considered significant. The array of invention, based on mobile phone, will be used to assess de-identified tissues or blood samples, and provide extra information to doctors about efficacies of drugs or radiations on patients.

As used herein, "consisting essentially of" allows the inclusion of materials or steps that do not materially affect the basic and novel characteristics of the claim. Any recitation herein of the term "comprising", particularly in a description of components of a composition or in a description of elements of a device, can be exchanged with "consisting essentially of" or "consisting of".

While the present invention has been described in conjunction with certain preferred embodiments, one of ordinary skill, after reading the foregoing specification, will be able to effect various changes, substitutions of equivalents, and other alterations to the compositions and methods set forth herein.

**Claims**

1. A single-cell array precursor comprising:

    a paper substrate;
    a polyanionic layer deposited on a surface of the paper substrate; and
    a patterned array of polycationic regions deposited on the polyanionic layer.

2. The precursor of claim 1, wherein the polyanionic layer comprises 3 to 5 layers of printing ink.

3. The precursor of claim 1 or claim 2, wherein the polycationic regions comprise polydiallyldimethyl ammonium chloride.

4. The precursor of any of the preceding claims, wherein the polycationic regions have a size of about 5 $\mu$m to about 40 $\mu$m.

5. A single-cell array comprising the precursor of any of the preceding claims and a plurality of cells individually attached to said polycationic regions.

6. The single cell array of claim 5, further comprising an agarose layer in which the cells are embedded.

7. A method of quantifying DNA damage in a plurality of individual cells, the method comprising:

    (a) contacting the single-cell array precursor of any of claims 1-4 with said plurality of individual cells, whereby individual cells are attached to polycationic regions of said precursor, thereby forming a single-cell array;
    (b) embedding the attached cells in a layer of agarose;
    (c) treating the cells with an alkaline solution to release their DNA into the agarose;
    (d) imaging the DNA released from each cell in the agarose; and
    (e) quantifying damaged DNA for each cell.

8. The method of claim 7, wherein the cells are exposed to a DNA-damaging agent prior to or during step (a).

9. The method of claim 7 or claim 8, wherein step (c) comprises exposing cells to a DNA-damaging agent.

10. The method of any of claims 7-9, wherein cells are imaged in step (d) using fluorescence microscopy, and optionally using a mobile phone camera as imaging device.

**11.** A method of predicting the efficacy of an anticancer treatment in a subject, the method comprising:

(a) providing the single cell array precursor of any of claims 1-4 and a plurality of cells from a subject;
(b) exposing the cells to an anticancer treatment;
(c) contacting the cells with the single cell array precursor; and
(d) quantifying DNA damage in the cells.

**12.** The method of any of claims 7-11, wherein DNA damage is quantified by automated determination of a nuclear diffusion factor.

**13.** A method of making the single cell array precursor of any of claims 1-4, the method comprising:

(a) providing a paper substrate, a polyanionic material, and a polycationic material;
(b) coating the paper with the polyanionic material to form one or more polyanionic layers on a side of the paper substrate; and
(c) coating regions of the polyanionic layer with the polycationic material to form an array of polycationic regions, the regions sized to allow attachment of a single cell to each polycationic region.

**14.** The method of claim 13, wherein step (b) comprises the use of an inkjet printer to print 3-5 layers of printing ink as the polyanionic material onto a surface of the paper substrate.

**15.** The method of claim 13, wherein step (c) comprises a microimprinting process.

**16.** A method of making a single cell array, the method comprising:

(a) providing the single cell array precursor of any of claims 1-4 and a plurality of single cells;
(b) contacting the polycationic regions of the single cell array precursor with a suspension comprising the plurality of single cells, whereby single cells from the suspension become attached to the polycation regions.

FIG. 1A    FIG. 1B

FIG. 2A  FIG. 2B  FIG. 2C  FIG. 2D  FIG. 2E  FIG. 2F

FIG. 3A  FIG. 3B  FIG. 3C  FIG. 3D
FIG. 3E  FIG. 3F  FIG. 3G  FIG. 3H

FIG. 4A

FIG. 4B

FIG. 4C

FIG. 4D

FIG. 4E

FIG. 4F

FIG. 5A

FIG. 5B

FIG. 5C

FIG. 5D

FIG. 5E

FIG. 5F

FIG. 6A
FIG. 6B
FIG. 6C
FIG. 6D
FIG. 6E
FIG. 6F

Camera phone

eyepiece

Emission filter

Objective lens

Cell array

Condenser lens

Excitation filter

Collector lens

UV LED

FIG. 7

Europäisches
Patentamt

European
Patent Office

Office européen
des brevets

# EUROPEAN SEARCH REPORT

Application Number

EP 17 15 9647

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GE LEI ET AL: "Lab-on-paper-based devices using chemiluminescence and electrogenerated chemiluminescence detection", ANALYTICAL AND BIOANALYTICAL CHEMISTRY, SPRINGER, DE, vol. 406, no. 23, 6 April 2014 (2014-04-06), pages 5613-5630, XP035377893, ISSN: 1618-2642, DOI: 10.1007/S00216-014-7756-1 [retrieved on 2014-04-06] * page 5614 * * page 5617 - page 5618 * * page 5623 - page 5624 * ----- | 1-16 | INV. B01J19/00 C12Q1/68 G01N33/50 |
| A | LIYUAN MA ET AL: "Predicting efficacies of anticancer drugs using single cell HaloChip assay", ANALYST, vol. 141, no. 8, 29 February 2016 (2016-02-29), pages 2454-2462, XP055404592, ISSN: 0003-2654, DOI: 10.1039/C5AN02564H * the whole document * ----- | 1-16 | |
| A | WO 2011/157895 A1 (AABO AKADEMI UNIVERSITY [FI]; IHALAINEN PETRI [FI]; MAEAETTAENEN ANNI) 22 December 2011 (2011-12-22) * claims 1-15 * ----- | 1-16 | TECHNICAL FIELDS SEARCHED (IPC) B01J C12Q G01N C12M |

The present search report has been drawn up for all claims

| Place of search | Date of completion of the search | Examiner |
|---|---|---|
| Berlin | 7 September 2017 | Cubas Alcaraz, Jose |

EPO FORM 1503 03.82 (P04C01)

## ANNEX TO THE EUROPEAN SEARCH REPORT
## ON EUROPEAN PATENT APPLICATION NO.

EP 17 15 9647

This annex lists the patent family members relating to the patent documents cited in the above-mentioned European search report.
The members are as contained in the European Patent Office EDP file on
The European Patent Office is in no way liable for these particulars which are merely given for the purpose of information.

07-09-2017

| Patent document cited in search report | Publication date | Patent family member(s) | Publication date |
|---|---|---|---|
| WO 2011157895 A1 | 22-12-2011 | NONE | |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62305122 A

**Non-patent literature cited in the description**

- **CARLSON.** *Biotechnol Healthc.,* September 2008, vol. 5 (3), 23-27